# EUROPEAN PATENT APPLICATION

(11) **EP 3 662 929 A1**
(43) Date of publication of application: **10.06.2020**
(21) Application number: 18211090.8
(22) Date of filing: 07.12.2018
(51) Int. Cl.: A61K 39/12

(54) **A RECOMBINANT KOI HERPESVIRUS (KHV) AND A DIVA VACCINE FOR PREVENTING AND/OR TREATING A DISEASE CAUSED BY KHV**

(71) Applicant: IDT Biologika GmbH, 06861 Dessau-Rosslau (DE)
(72) Inventor: FUCHS, Walter, 17493 Greifswald (DE); SCHRÖDER, Lars, 17493 Greisfwald (DE); BERGMANN, Sven M., 17493 Greisfwald (DE); KLAFACK, Sandro, 17493 Greifswald (DE); FICHTNER, Dieter, 17493 Greisfwald (DE); DAUBER, Malte, 17493 Greisfwald (DE); LEE, Pei-Yu Alison, 407 Taichung (TW)
(74) Representative: Isarpatent

(57) **Abstract**

The present invention relates to a recombinant KHV with a deficient ORF148/149, a recombinant KHV DNA encoding said KHV, a DIVA vaccine comprising said KHV and/or said DNA for preventing and/or treating disease caused by KHV in fish, such as *Cyprinus carpio*, a eukaryotic cell comprising said KHV and/or said DNA, a fish, such as *Cyprinus carpio*, vaccinated with said vaccine, and a method for preparing infectious particles of said KHV.

## Description

### Technical field

The present invention relates to a recombinant Koi herpesvirus (KHV) and a vaccine comprising the same for preventing or treating a disease caused by Koi herpesvirus in *Cyprinus carpio.* Furthermore, the present invention relates to a method wherein vaccinated fish can be differentiated from those infected by wild-type KHV.

### Background

Koi herpesvirus (KHV) also named cyprinid herpesvirus 3 (CyHV-3) is a pathogen capable of inducing a severe and acute disease in common carp (*Cyprinus carpio carpio*) causing high mortalities of up to 70% or more. Common carps constitute one of the largest volumes of farmed fish worldwide, and KHV is a globally significant disease notifiable to the World Organisation for Animal Health (OIE). The virus also affects carp varieties such as koi carp (*Cyprinus carpio koi*) and ghost carp and hybrids of common carp, e.g. goldfish x common carp. In addition, PCR detection of KHV performed on cyprinid and non-cyprinid fish species, but also on freshwater mussels and crustaceans, suggested that these species could act as reservoirs of the virus. This makes disease control complex and challenging.

One key feature of KHV infection is the presence of persistently infected fish that contribute to the circulation of the disease in water bodies that have experienced KHV outbreaks. PCR and seroprevalence studies in such water bodies indicate a high number of positive fish more than 2 years after a major outbreak. KHV has been reported in more than 25 countries in Asia, Europe, Africa as well as US and Canada. Due to the global spread of this disease and the impact on carp production, KHV disease is an OIE notifiable disease and a regulated disease with national disease control programs in some countries (E.g. Germany, UK). The nature of this disease is such that it is likely that the virus is present in many more countries, but has not yet been identified or reported.

According to FAO, farmed carp (grass, silver and common carp) accounted for 37.5% of global aquaculture production in 2015 with 28.8 million tons. This is equivalent to 25.6% of the total value of all aquaculture animals in 2015. The average production growth between 1996 and 2015 was 5.3%. Common carp production was 4.3 million tons in 2015.

Asia is the largest producer of common carp by region in 2012. Key producing countries are China, Indonesia, Vietnam and Bangladesh. KHV is amongst others present in Indonesia, Japan, Malaysia and Thailand. China is by far the largest producer of common carp, accounting for 2.9 million tons in 2012. KHV was reported in China in 2002. Since then, it has been reported in several provinces. It is possible that some other Asian countries do not report KHV even if present due to lack of surveillance and unwillingness to report due to trade consequences. The second largest production region is Eastern Europe where carp is typically cultured as a festive dish, and the production predominantly occurs in the Russian Federation, Ukraine, Czech Republic and Poland but also in Germany and France.

Because of such economic relevance of disease caused by KHV, as explained above, vaccines which are safe, efficacious and affordable are urgently needed. In this context, a principle of Differentiating Infected from Vaccinated Animals (DIVA) is mandatory in many countries. In this regard, several inactivated KHV preparations and attenuated live virus vaccines obtained after cell culture passage and/or UV irradiation of virulent KHV have been demonstrated to be useful (Ronen et al., 2003, Perelberg et al., 2005). However, such vaccines fail to be used as DIVA vaccine, since the vaccinated fish is not distinguishable from those naturally infected fish, which largely restricts the use of said vaccine.

In this regard, it has been noticed that the genome of KHV, i.e. CyHV-3 genome, contains approximately 155 open reading frames (ORFs) predicted to encode proteins (Aoki et al., 2007). Analysis of purified virus particles by mass spectrometry identified 40 different CyHV-3 encoded proteins, including 3 capsid components, and 13 membrane proteins (Michel et al., 2010). The integral membrane proteins pORF25, pORF65, pORF81, pORF99, pORF136, pORF146, pORF148 and pORF149 were identified and characterized by specific antisera or monoclonal antibodies (mAb), and, except pORF81 and pORF136, proved to be modified by N-linked glycosylation (Rosenkranz et al., 2008; Vrancken et al., 2013; Fuchs et al., 2014). ORFs 25, 65, 148, and 149 constitute members of a family of distantly related genes which is conserved in cyprinid herpesviruses, and might have evolved from a common ancestor (Andrew J. Davison et al., 2013; Aoki et al., 2007). Indirect immunofluorescence (IF) tests of eukaryotic cells transfected with expression plasmids for ORF25, ORF65, ORF99, ORF148 or ORF149 revealed specific reactions of the respective glycoproteins with serum antibodies from experimentally and naturally KHV-infected carp (Fuchs et al., 2014). Therefore, vaccines based on KHV mutants lacking these genes might be suitable for serological differentiation between infected and vaccinated animals (DIVA) (van Oirschot, 1999).

On the other hand, candidate subunit or DNA vaccines containing or encoding single CyHV-3 envelope proteins like pORF25 or pORF81 have been shown to induce protective immune responses (Cui et al., 2015; Zhou et al., 2014a; Zhou et al., 2014b), suggesting that their deletion might affect the protective efficacy of CyHV-3 live vaccines. Recent studies demonstrated that the four ORF25 family genes including ORF65, ORF148, and ORF149 are not essential for CyHV-3 replication in cell culture, and that deletion of ORF25 attenuated the virus *in vivo,* but significantly reduced its protective efficacy against wild-type challenge (Vancsok et al., 2017). In contrast, single deletions of ORF65, ORF148, or ORF149 did not lead to sufficient attenuation of a virulent CyHV-3 strain in carp (Vancsok et al., 2017).

Therefore, given the complex functions of ORFs in KHV, a thorough inventive research is urgently needed to provide an improved vaccine, suitable for mass application, which supports DIVA principle to differentiate naturally infected from vaccinated fishes, and meanwhile can sufficiently provide a fish, at least a common carp, prevention or treatment of disease caused by KHV.

### Brief description of the invention

The first aspect of the present invention is to provide a recombinant KHV in which the ORFs 148 and 149 (ORF148/149) coding for immunogenic envelope glycoproteins are deficient. The deficiency of genes ORF148/149 coding for such antibody-inducing glycoproteins allows differention of vaccinated from wild-type virus infected carps through DIVA test.

Against ORF148 and/or 149 antibody may be used for establishment of an ELISA for DIVA. However, other antibodies which can differentiate the blood serum of fish vaccinated with the presently disclosed recombinant KHV with deficiency of ORF148/149 from those infected with wild-type KHV may also be used. The term "antibody" encompasses various forms of antibodies, preferably monoclonal antibodies including, but not being limited to, whole antibodies, antibody fragments, chimeric antibodies, and genetically engineered antibodies (variant or mutant antibodies) as long as the characteristic properties according to the invention are retained.

In a preferred embodiment, one or more of ORFs 25, 65, 92, 99, 136, 138, 146 (immunogenic envelope proteins), ORF56, ORF57, ORF55 (thymidine kinase), and ORF123 (deoxyuridine triphosphatase) are further additionally deficient. Deficiency of ORFs 55 and 123 (ORF55/123) may disable the ability of the virus to cause clinical disease in vaccinated fish.

As used herein, "deficient" ORF means that the ORF is no longer functional, i.e. no longer capable of encoding a functional protein. Such deficiency may be obtained by mutations such as deletion, replacement, or insertion one or more nucleotides in the genes encoding said ORFs, or in their promoter regions. Such mutation may be a frame shift mutation at the 5' site of the genes, a complete deletion or partial deletion of the promoter region, or a complete deletion or partial deletion of the genes themselves.

As used herein, the nucleotide can be a cDNA molecule, or fragment thereof. The nucleotides can be desoxyribonucleotides, ribonucleotides, modified nucleotides or bases, and/or their analogs, or any substrate that can be incorporated into a polymer by DNA or by a synthetic reaction. Said nucleotides may comprise modified nucleotides, such as methylated nucleotides and their analogs. The modification to the nucleotide structure may be imparted before or after assembly of the polymer. The sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (*e.g*., methyl phosphonates, phosphotriesters, phosphoamidates, carbamates, *etc.*) and with charged linkages (*e.g.,* phosphorothioates, phosphorodithioates, *etc.*), those containing pendant moieties, such as, for example, proteins (*e.g.,* nucleases, toxins, antibodies, signal peptides, ply-L-lysine, *etc*.), those with intercalators (*e.g.,* acridine, psoralen, *etc.*), those containing chelators (*e.g.,* metals, radioactive metals, boron, oxidative metals, *etc.*), those containing alkylators, those with modified linkages (*e.g.,* alpha anomeric nucleic acids, *etc*.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Nucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR₂ ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical.

In a preferred embodiment, the whole genes encoding ORFs 55, 123, 148 and 149 (ORFs 55/123/148/149) are deleted in said recombinant KHV, which results in no production of ORFs 55/123/148/149 proteins at all and leads to attenuation of KHV.

Said recombinant KHV in the present invention may be in a live form. More preferably, said KHV is able to reconstitute infectious particles, namely to replicate when introduced into eukaryotic cells or fish individuals, wherein such fish individuals are preferably *Cyprinus carpio,* more preferably, *Cyprinus carpio carpio* and/or *Cyprinus carpio koi.*

In another preferred embodiment, said recombinant KHV in the present invention is additionally deficient in one or more genes which is/are essential for replication, or deficient in one or more genes which is/are essential for virulence but are not essential for replication, which renders said recombinant KHV inactive.

Preferably, the recombinant KHV according to the present invention is in a non-replicative form. A "non-replicative form" used herein means that the recombinant KHV is still able to infect cells or fish individuals, e.g. *Cyprinus carpio, Cyprinus carpio carpio* or *Cyprinus carpio koi,* but is not able to replicate to the level that infective progeny virus is formed. A non-replicative recombinant strain can be produced by inactivation of a KHV gene that is essential for replication, through regular techniques such as insertion, deletion or mutation, e.g. using BAC cloning. Said virus in a non-replicative form can be cultured on a permissive cell line stably expressing said inactivated gene.

Any gene which contributes to replication can be inactivated in order to obtain a KHV in a non-replicative form. Gene(s) of which the inactivation leads to a KHV in a non-replicative form, may be deleted. Preferably, a gene of the recombinant KHV in the present invention that is deleted in order to give rise to a KHV in a non-replicative form, is selected from the group consisting of: ORF25, ORF31, ORF32, ORF34, ORF35, ORF42, ORF43, ORF45, ORF51, ORF59, ORF60, ORF62, ORF65, ORF66, ORF68, ORF70, ORF72, ORF78, ORF81, ORF84, ORF89, ORF90, ORF92, ORF95, ORF97, ORF99, ORF108, ORF115, ORF131, ORF132, ORF136, ORF137, ORF148 and ORF149.

The recombinant KHV in the present invention may comprise a bacterial artificial chromosome (BAC) vector sequence which is well known in the art for being used in the construction of recombinant virus such as herpesviruses. Such BAC vector sequence can be inserted in any viral gene which is essential for virulence and or any viral gene which is or isn't essential for replication and/or any intergenic regions.

In a preferred embodiment, the recombinant KHV in the present invention comprises a heterologous DNA fragment, e.g. a heterologous gene. Such a heterologous DNA fragment may be a heterologous gene encoding an immunogenic protein of another virus or microorganism that is pathogenic to fish, preferably *Cyprinus carpio,* more preferably *Cyprinus carpio carpio* and/or *Cyprinus carpio koi.* Said heterologous gene may be the G glycoprotein of rhabdovirus causing spring viraemia of carp. Such construct comprising said heterologous not only protects the carp from KHV but also from spring viraemia of carp, when used in a vaccine. The promoter for the expression of a heterologous gene, e.g. the G glycoprotein of rhabdovirus, may be the HCMV IE promoter.

The second aspect of the present invention is to provide a recombinant KHV DNA which encodes the presently disclosed recombinant KHV comprising the feature(s) disclosed above.

The third aspect of the present invention is to provide a recombinant KHV comprising the feature(s) disclosed above and/or a recombinant KHV DNA encoding such recombinant KHV for use as a medicament. Particularly, the present inventions provides a recombinant KHV comprising the features disclosed above and/or a recombinant KHV DNA encoding such recombinant KHV for use in a method of preventing and/or treating a disease in fish caused by KHV, wherein the fish is preferably *Cyprinus carpio,* more preferably *Cyprinus carpio carpio* and/or *Cyprinus carpio koi.*

As used herein, "treatment", "treat" or "treating" refers to clinical intervention in an attempt to alter the natural course of the individual or cell being treated. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. Such term also refers to improving, alleviating, and/or decreasing the severity of one or more symptoms of a condition being treated.

Because of the attenuation of KHV in the present invention, the recombinant KHV disclosed above and/or a recombinant KHV DNA encoding such recombinant KHV may be suitable for the immunization of fish, preferably *Cyprinus carpio,* more preferably *Cyprinus carpio carpio* and/or *Cyprinus carpio koi.* Such immunization may be used in a method of preventing or treating a disease of fish caused by KHV, wherein the fish is *Cyprinus carpio,* preferably *Cyprinus carpio carpio* and/or *Cyprinus carpio koi.*

The fourth aspect of the present invention is to provide a vaccine comprising the recombinant KHV disclosed above and/or a recombinant KHV DNA encoding such recombinant KHV, and preferably a pharmaceutically acceptable adjuvant. The term "vaccine" used herein refers to a composition which is able to prevent a host from a particular disease and/or therapeutically treat a host suffered from to a particular disease. Said vaccine may produce prophylactic or therapeutic immunity. Said vaccine may further comprise stabilizers.

The fifth aspect of the present invention to provide said vaccine as disclosed above for use in a method of preventing and/or treating a disease in fish caused by KHV, wherein the fish is preferably *Cyprinus carpio,* more preferably *Cyprinus carpio carpio* and/or *Cyprinus carpio koi.*

Preferably, said vaccine comprising the recombinant KHV disclosed above and/or a recombinant KHV DNA encoding such recombinant KHV, also carries the gene encoding G glycoprotein of rhabdovirus causing spring viraemia of carp, and preferably a pharmaceutically acceptable carrier, for preventing and/or treating a disease in fish caused by a rhabdovirus causing spring viraemia of carp.

Vaccines in the present invention may be prepared as liquid solutions, emulsions or suspensions for injection or delivery through immersion of fish in water. A liquid solution concentrate can be prepared for being added to a water tank or bath. The vaccine in the present invention may be also in a dry form, e.g. powder, lyophilized, in a compressed pellet or tablet form. Solid form, e.g. powder, which is suitable for dissolution in, or suspension in, liquid vehicles or for mixing with solid food, may be prepared before administration. The vaccine may be a lyophilized culture in a ready-to-use form for reconstitution with a sterile diluent. Lyophilized cells may be reconstituted in 0.9% saline. A formulation of injectable vaccine may be an emulsion. Liquid or reconstituted forms of said vaccine may be diluted in a small volume of water (e.g. 1:100 to 1:20,000) before being added to a tank or bath.

In the present invention, said KHV may be in the form of a tissue culture fluid. Said fluid may be stored at the ambience, preferably at -70 to -20 °C, most preferably as a solution containing gelatine and glucose.

The recombinant KHV disclosed in the present invention may be converted into a dry form by several methods, e.g. lyophilization. Before drying, different ingredients such as preservatives, anti-oxidants or reducing agents, a variety of excipients, may be added to the medium. Such excipients may also be added to said KHV after the drying step. If said vaccine in the present invention is used for oral administration, e.g. through dipping or balneation, administration of an adjuvant may be not needed. However, if said vaccine is injected directly into the fish, an adjuvant may be used. If the recombinant KHV according to the present invention is in a non-replicating form, immune stimulants may be added.

In order to boost the immune response, said vaccine in the present invention may include a variety of adjuvants, cytokines or other immune stimulants, particularly in the case of vaccine that is intended for injection.

An adjuvant is an immunostimulatory substance boosting the immune response of the host in a non-specific manner. The adjuvant may be hydrophilic adjuvant, e.g. aluminum hydroxide or aluminum phosphate, or hydrophobic adjuvant, e.g. mineral oil based adjuvants. Adjuvants such as muramyl dipeptides, avidine, aluminium hydroxide, aluminium phosphate, oils, oil emulsions, saponins, dextran sulphate, glucans, cytokines, block co-polymers, immunostimulatory oligonucleotides and others known in the art may be mixed with the recombinant KHV according to the invention. Examples of adjuvants frequently used in fish farming are muramyldipeptides, lipopolysaccharides, several glucans and glycans and Carbopol® (homopolymer). Suitable adjuvants are e.g. water in oil (w/o) emulsions, o/w emulsions and w/o/w double-emulsions. Oil adjuvants suitable for use in w/o emulsions are e.g. mineral oils or metabolisable oils. Mineral oils are e.g. Bayol®, Marcol® and Drakeol®; metabolisable oils are e.g. vegetable oils, such as peanut oil and soybean oil, or animal oils such as the fish oils squalane and squalene. Alternatively a vitamin E (tocopherol) solubilisate as described in EP 382,271 may be used. Suitable o/w emulsions are e.g. obtained starting from 5-50 % w/w water phase and 95-50 % w/w oil adjuvant, more preferably 20-50 % w/w water phase and 80-50 % w/w oil adjuvant are used.

In a preferred embodiment of the present invention, said vaccine may additionally comprise a stabilizer. A stabilizer can be added to a vaccine according to the invention to protect it from degradation, to enhance the shelf-life, or to improve freeze-drying efficiency. Regular stabilizers are SPGA (Bovarnik et al., 1950, J. Bacteriology, vol. 59, p. 509), skimmed milk, gelatine, bovine serum albumin, carbohydrates e.g. sorbitol, mannitol, trehalose, starch, sucrose, dextran or glucose, lactoses, proteins such as albumin or casein or degradation products (arginine) thereof, and buffers, such as alkali metal phosphates. Antibiotics such as neomycin and streptomycin may be added to prevent the potential growth of germs. In addition, said vaccine in the present invention may comprise one or more suitable surface-active compounds or emulsifiers, e.g. Span ® or Tween®. Said vaccine in the present invention may also comprise a "vehicle". The term "vehicle" refers to a compound to which the KHV (either in form of a virus particle or in form of DNA) in the present invention adheres, without being covalently bound to it. Such vehicles may be bio-microcapsules, micro -alginates, liposomes or macrosols. A special form of such a vehicle may be an ISCOM.

In the present invention, said vaccine may be administered to fish individually-orally, e.g. through their feed or by forced oral administration, or by injection, e.g. via the intramuscular or intraperitoneal route. Alternatively the vaccine may be administered simultaneously to the entire fish population contained in a body of water by spraying, dissolving and/or immersing the vaccine or by feed.

In a preferred embodiment, said vaccine in the present invention is contained in the feed of fish, and said fish is vaccinated by eating the feed containing said vaccine.

These methods are useful for vaccination of all kinds of fish, and in various environments such as ponds, aquariums, natural habitat and fresh water reservoirs.

In the present invention, said vaccine may comprise DNA encoding said recombinant KHV. DNA vaccines in the present invention comprise the genome of said recombinant KHV according to the present invention. They may be administered through intradermal application e.g. using a needle-less injector such as a GeneGun®. This way of administration delivers the DNA directly into the cells of fish to be vaccinated. A preferred amount of a recombinant KHV DNA in a pharmaceutical composition according to the present invention is in the range between 10 pg and 1000 µg. Preferably, amounts in the range between 0.1 and 100 µg are used. Alternatively, fish can be immersed in solutions comprising e.g. between 10 pg and 1000 µg/ml of the DNA to be administered. All these techniques and routes of administration are well-known in the art. Preferably the vaccine according to the invention is formulated in a form suitable for injection or for immersion vaccination, such as a suspension, solution, dispersion, emulsion, and the like.

The dosing scheme for the application of a vaccine according to the invention to the target fish can be the application of single or multiple doses, which may be given at the same time or sequentially, in a manner compatible with the dosage and formulation and in such an amount as will be immunologically effective. Whether a treatment is "immunologically effective" may be determined, e.g. by administering an experimental challenge infection to vaccinated fish, and next determining the clinical signs of disease and mortality due to the disease, serological parameters, or by measuring re-isolation of the pathogen in the target fish.

A preferred amount of a live vaccine comprising recombinant KHV according to the invention is expressed for instance as plaque forming units (pfu). For instance for a live viral vector a dose range between 1 and 10¹⁰ plaque forming units (pfu) per animal dose may advantageously be used; preferably a range between 10¹ and 10⁶ pfu/dose.

If a vaccine comprises a non-replicative form of the recombinant KHV according to the invention, the dose would be expressed as the number of non-replicative virus particles to be administered. Then dose would usually be somewhat higher when compared to the administration of live virus particles, because live virus particles replicate to a certain extent in the target animal, before they are removed by the immune system. For vaccines on the basis of non-replicative virus particles, an amount of virus particles in the range of about 10⁴ to 10⁹ particles would usually be suitable.

Preferably, the vaccine in the present invention is administered via immersion/bath, especially when a live recombinant KHV according to the invention is used. The vaccine in the present invention may also be used for parenteral vaccination.

The sixth aspect of the present invention is to provide a eukaryotic cell comprising the recombinant KHV and/or the recombinant KHV DNA as disclose above.

The seventh aspect of the present invention is to provide a fish which has been vaccinated with a vaccine according to the fourth aspect of the present invention, wherein said fish is preferably *Cyprinus carpio,* more preferably *Cyprinus carpio carpio* and/or *Cyprinus capio koi.*

The eighth aspect of the present invention it to provide a method for preparing infectious particles of recombinant KHV, wherein said method comprises the steps of: (i) introducing a recombinant KHV or a recombinant KHV DNA according to the present invention, as disclosed above, into permissive eukaryotic cells; and (ii) culturing said eukaryotic cells to produce recombinant KHV.

### Examples

The present invention will now be described in detail with reference to examples thereof. However, these examples are illustrative and do not limit the scope of the invention.

### Example 1 Generation and genomic characterization of a recombinant KHV

Genomic KHV-T (cell culture adapted strain from Taiwan, see also Schröder et al., 2018) DNA was prepared from infected cell lysates as described (Fuchs and Mettenleiter, 1996). For the generation of deletion mutants, transfer plasmids were prepared after amplification of the targeted glycoprotein gene regions from KHV-T DNA by PCR using Pfx DNA polymerase (Thermo Fisher Scientific). Double-deletion of ORF55/123 (ΔORF55/123) is generated through regular homologues recombination techniques. For comparison, single deletion of ORF55 (ΔORF55) and ORF 123 (ΔORF123) were also generated. With the same techniques ORF148/149 encoding antibody-inducing glycoproteins were deleted in the ΔORF55/123 strain (ΔORF55/123/148/149). Genomic DNA of all generated CyHV-3 recombinants was analyzed by restriction and Southern blot analyses, and the modified genome regions were further characterized by PCR and sequencing using the specific primers.

KHV-T and its derivatives were propagated in Common Carp Brain (CCB) cells (Neukirch et al., 1999), which were grown in minimum essential medium (MEM) supplemented with 10 % fetal bovine serum (FBS) at 25°C. Infected cells were incubated in medium containing only 5 % FBS, and, after appearance of cytopathogenic effects, lysed by freeze-thawing and stored in aliquots at -80°C. For plaque assays, CCB cell monolayers were incubated with serial virus dilutions for 2 h. Subsequently, the inoculum was replaced by MEM containing 5 % FBS and 6 g/l of methylcellulose, and incubation at 25°C was continued for 3 to 7 days.

The production process is based on the propagation in cell culture vials and/or bioreactors. Obtained yields reach 6-7 log₁₀ pfu/ml KHV-TΔORF55/123/148/149 mutant prior to process optimization. No difference in yields, growth characteristic and plaque size was observed between KHV-TΔORF55/123/148/149 mutant and parental KHV-T without gene deletions. The propagated virus is stabilized before freezing and storage at ≤ -20°C. The vaccine is diluted in freshwater before use.

### Example 2 Attenuation and protective efficacy of the recombinant KHV

*In vivo* attenuation and protective efficacy of the generated ΔORF55, ΔORF123 and ΔORF55/123 mutants of KHV-T was tested using five groups of carp, each consisting of 40 nine months-old (approximately 8 g / 5-7 cm) and 5 two years-old (approximately 200 g / 20-23 cm) fish (Fischzucht Traßdorf 1967 e.V., TraBdorf, Germany). The animals were kept at a constant temperature of 20°C, and infected by immersion for 1 h in 5 I water containing 2.5 x 10⁷ PFU of either KHV-T, KHV-TΔORF55, KHV-TΔORF123, KHV-TΔORF55/123, or cell culture medium. After 35 d, all carp that had survived primary CyHV-3 infection were challenged by immersion for 1 h in 5 I water containing 2.5 x 10⁷ PFU of KHV-T. The fish were examined for additional 28 days for clinical symptoms. Blood samples and gill swabs were taken from the two years-old carp before, and two and four weeks after primary infection, as well as after challenge. The young carps were considered for evaluation of the mortality rates. Statistical significance of differences between the groups was evaluated using two-sided Fisher's exact tests.

Such ΔORF55/123 double-deletion mutant showed superior safety and equal efficacy compared to mutant strains containing a single deletion of either gene encoding ORF55 (ΔORF55) or 123 (ΔORF123).

The prototype vaccine has been shown to confer complete protection from homologues and heterologous challenge 42 days post vaccination in carps after a 30 min vaccine-immersion bath at 20°C containing 5x10³ pfu/ml of the ΔORF55/123/148/149 mutant, wherein the challenge was conducted with 5x10⁷ pfu/10l KHV-T and 5x10⁶ pfu/10l KHV isolates from Israel (KHV-I).

After vaccination of carps (30 min immersion bath at 20°C containing 5x10³ pfu/ml of the ΔORF55/123/148/149 mutant) none of the fish showed neither morbity nor mortality. Thus application of the vaccine is considered to be safe.

### Example 3 Differentiating vaccinated fish from those infected with wild-type KHV (DIVA test)

Enzyme-linked immunosorbent assay (ELISA) was applied for DIVA test by using anti-ORF149 monoclonal antibodies. Blood samples from vaccinated (ΔORF55/123/148/149) and infected carps were centrifuged at 600 x g for 15 min and the supernatant was used in the ELISA. NUNC POLYSORP immuno-plates (Thermo Fisher Scientific) were coated with sucrose-gradient purified KHV-T particles, blocked, incubated with the 1:300 diluted sera in two replicates. An anti-ORF149 monoclonal antibody and horseradish peroxidase-conjugated anti-mouse IgG (Jackson ImmunoResearch) were added. Substrate reactions were measured in an Infinite F200 microplate reader (Tecan), and OD₄₅₀ₙₘ values > 0.2 were considered as positive.

The results revealed that further deletion of genes ORF148/149 coding for antibody-inducing glycoproteins allows differentiation of vaccinated from wildtype virus infected carps through DIVA test.

### Conclusions

In the present invention, the recombinant KHV being deficient in ORF148/149 coding for antibody-inducing glycoproteins allows differentiation of vaccinated from wildtype virus infected carps, which therefore supports DIVA principle. Thus the present invention displays an advantage for its application when DIVA principle is mandatory. When the presently disclosed recombinant KHV is further modified, e.g. with additional deletion of ORF55/123, the ability of the virus to cause clinical disease in vaccinated fish would be significantly disabled. Therefore, with the present invention, a vaccine which is safe, efficacious and affordable for preventing or treating fish disease caused by KHV can be provided especially to those countries where vaccinated fish, particularly vaccinated *Cyprinus carpio,* preferably vaccinated *Cyprinus carpio carpio* and/or *Cyprinus carpio koi* has to be differentiated from those infected with wild-type KHV.

## Claims

1. A recombinant Koi herpesvirus (KHV), wherein Open Reading Frames (ORFs) 148 and 149 (immunogenic envelope glycoproteins) are deficient.

2. The recombinant KHV according to claim 1, wherein one or more of ORFs 25, 65, 92, 99, 136, 138, 146 (immunogenic envelope proteins), ORF56, ORF57, ORF55 (thymidine kinase), and ORF123 (deoxyuridine triphosphatase) are additionally deficient.

3. The recombinant KHV according to claim 1 or 2, wherein ORFs 55, 123, 148 and 149 are deficient.

4. The recombinant KHV according to any of claims 1-3, wherein the deficient ORFs are obtained by mutations such as deletion, replacement, or insertion of one or more nucleotides in the genes encoding said ORFs, or in their promoter regions, or by mutations such as a frame shift mutation at the 5' site of the genes, a complete deletion or partial deletion of the promoter region, or a complete deletion or partial deletion of the genes themselves.

5. The recombinant KHV according to any of claims 1-4, wherein the herpesvirus is in a live form or in a non-replicative form.

6. The recombinant KHV according to any of claims 1-5, further comprising a heterologous DNA fragment.

7. A recombinant KHV DNA encoding the recombinant KHV according to any of claims 1-6.

8. A recombinant KHV according to any of claims 1-6 and/or a recombinant KHV DNA according to claim 7 for use as a medicament.

9. A recombinant KHV according to any one of claims 1-6 and/or a recombinant KHV DNA according to claim 7, for use in a method of preventing and/or treating a disease in fish caused by KHV, wherein the fish is preferably *Cyprinus carpio,* more preferably *Cyprinus carpio carpio* and/or *Cyprinus carpio koi.*

10. A vaccine comprising a recombinant KHV according to any of claims 1-6 and/or a recombinant KHV DNA according to claim 7, and preferably a pharmaceutically acceptable adjuvant.

11. A vaccine for use in a method of preventing and/or treating a disease in fish caused by KHV, wherein said vaccine comprises a recombinant KHV according to any of claims 1-6 and/or a recombinant KHV DNA according to claim 7, and a pharmaceutically acceptable adjuvant, wherein the fish is preferably *Cyprinus carpio,* more preferably *Cyprinus carpio carpio* and/or *Cyprinus carpio koi.*

12. The vaccine for use according to claim 11, wherein the vaccine is used for immersion/bath vaccination or parenteral vaccination.

13. A eukaryotic cell comprising the recombinant KHV according to any of claims 1-6 and/or the recombinant KHV DNA according to claim 7.

14. A fish, in particular *Cyprinus carpio,* preferably *Cyprinus carpio carpio* and/or *Cyprinus carpio koi,* which has been vaccinated with a vaccine according to claim 10.

15. A method for preparing infectious particles of recombinant KHV, wherein said method comprises the steps of:
(i) introducing a recombinant KHV according to any of claims 1-6 or a recombinant KHV DNA according to claim 7 into permissive eukaryotic cells; and
(ii) culturing said eukaryotic cells to produce recombinant KHV.
